(19) Europäisches Patentamt
European Patent Office
Office européen des brevets

(11) EP 1 056 711 B1

(12) EUROPÄISCHE PATENTSCHRIFT

(45) Veröffentlichungstag und Bekanntmachung des Hinweises auf die Patenterteilung:
**06.10.2004 Patentblatt 2004/41**

(21) Anmeldenummer: **98967117.7**

(22) Anmeldetag: **23.07.1998**

(51) Int Cl.[7]: **C07C 67/62**, C07C 69/54

(86) Internationale Anmeldenummer:
**PCT/EP1998/004631**

(87) Internationale Veröffentlichungsnummer:
**WO 1999/007664 (18.02.1999 Gazette 1999/07)**

# (54) VERFAHREN ZUR STABILISIERUNG VON (METH)ACRYLSÄUREESTERN GEGEN UNERWÜNSCHTE RADIKALISCHE POLYMERISATION

PROCESS FOR STABILISING (METHA)ACRYLIC ESTERS AGAINST UNWANTED RADICAL POLYMERISATION

PROCEDE DE STABILISATION D'ESTERS D'ACIDE (METH)ACRYLIQUE VIS-A-VIS D'UNE POLYMERISATION PAR VOIE RADICALAIRE INDESIRABLE

(84) Benannte Vertragsstaaten:
**BE DE DK ES FR GB IT NL**

(30) Priorität: **07.08.1997 DE 19734171**
**15.08.1997 DE 19735223**

(43) Veröffentlichungstag der Anmeldung:
**06.12.2000 Patentblatt 2000/49**

(73) Patentinhaber: **BASF AKTIENGESELLSCHAFT**
**67056 Ludwigshafen (DE)**

(72) Erfinder:
- **AICHINGER, Heinrich**
**68199 Mannheim (DE)**
- **HERBST, Holger**
**67227 Frankenthal (DE)**
- **NESTLER, Gerhard**
**67071 Ludwigshafen (DE)**

(56) Entgegenhaltungen:
**EP-A- 0 522 709** **EP-A- 0 791 573**

- **DATABASE WPI Week 9402 Derwent Publications Ltd., London, GB; AN 94-012301 XP002085021 & JP 05 320217 A (KURURAYCO LTD) , 3. Dezember 1993 in der Anmeldung erwähnt**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 056 711 B1

Printed by Jouve, 75001 PARIS (FR)

## Beschreibung

Vorliegende Erfindung betrifft ein Verfahren zur Stabilisierung von als Reinsubstanz oder als Bestandteil von Gemischen, die keine Brönsted-Säure (keine protische Säure) enthalten deren Brönsted-Säurestärke größer als die Brönsted-Säurestärke von Acrylsäure ist, vorliegenden Estern der (Meth)acrylsäure gegen unerwünschte radikalische Polymerisation durch Zusatz eines ein p-Phenylendiamin enthaltenden Polymerisationsinhibitors.

(Meth)acrylsäure wird in dieser Schrift als verkürzte Schreibweise für "Acrylsäure und/oder Methacrylsäure" verwendet.

Ester der (Meth)acrylsäure verfügen aufgrund ihrer aktivierten Vinylgruppe über eine ausgeprägte Neigung zur radikalischen Polymerisation. Dies ist insofern von Vorteil, als sich (Meth)acrylate dadurch in hervorragender Weise zur gezielten Herstellung von Polymerisaten auf dem Weg der initiierten radikalischen Polymerisation eignen, wie sie z.B. zur Produktion von Klebstoffen benötigt werden. Gleichzeitig ist die ausgeprägte Neigung zur radikalischen Polymerisation aber insofern von Nachteil, als es sowohl bei der Lagerung als auch bei der chemischen und/oder physikalischen Bearbeitung (z.B. Destillation oder Rektifikation) der (Meth)acrylate oder solche Ester enthaltender Gemische, insbesondere unter der Einwirkung von Wärme und/oder Licht, zur unerwünschten, spontanen, radikalischen Polymerisation der Ester der (Meth)acrylsäure kommen kann. Abgesehen davon, daß derartige unkontrollierte radikalische Polymerisationen der (Meth)acrylate oder sie enthaltender Gemische ein erhebliches Gefahrenpotential beinhalten (die radikalische Polymerisation verläuft stark exotherm und als Konsequenz häufig explosionsartig), ziehen sie darüber hinaus vielfältige andere Folgewirkungen nach sich. Beispielsweise kann sich bei der Destillation von (Meth)acrylsäureester enthaltenden Gemischen in unerwünschter Weise gebildetes Polymerisat auf der Oberfläche des Verdampfers niederschlagen (dort ist die Neigung zur Polymerisatbildung infolge der hohen Temperaturen erhöht) und dadurch den Wärmeübergang in unerwünschter Weise mindern. Gebildetes Polymerisat kann aber auch die Einbauten in Rektifikationskolonnen verstopfen, was unerwünschte Druckverluste verursacht. Beides erfordert das Unterbrechen des Rektifikationsprozesses, um in aufwendiger Weise das gebildete Polymerisat zu entfernen.

Es ist daher allgemeine Praxis, sowohl Estern der (Meth)acrylsäure als auch solche Ester enthaltenden Gemischen sowohl bei der Lagerung als auch bei der chemischen und/oder physikalischen Bearbeitung Verbindungen zuzusetzen, die als Inhibitoren einer radikalischen Polymerisation der (Meth)acrylate wirken und dadurch die (Meth) acrylsäureester gegen unerwünschte radikalische Polymerisation stabilisieren.

Aus der WO 92/01665 ist bekannt, als solche Inhibitoren ein Gemisch aus in bestimmter Weise N,N-substituierten p-Phenylendiaminen einzusetzen. Dabei lehrt die WO 92/01665, daß solche p-Phenylendiamine insbesondere im Beisein starker Säuren günstige Polymerisationsinhibitoren sind. Die US-A 4,797,504 empfiehlt, p-Phenylendiamine in Kombination mit Hydroxylaminen als Polymerisationsinhibitoren für (Meth)acrylsäureester zu verwenden.

Die GB-A 1.064,845 lehrt ganz generell, Monomeren die eine Vinylgruppe aufweisen, als Polymerisationsinhibitoren organische Verbindungen zuzusetzen, die eine Nitroso-Gruppe, d. h., eine Atomgruppierung -N=O, aufweisen.

Aus der US-A 5,322,960 ist bekannt, Estern der (Meth)acrylsäure als Polymerisationsinhibitoren ein Gemisch zuzusetzen, das u. a. ein Nitroxyl-Radikal (eine Verbindung, die wenigstens eine >N-O- Gruppe aufweist) enthält.

Die JP-A 5/320217 lehrt u. a., (Meth)acrylsäure gegen unerwünschte radikalische Polymerisation dadurch zu stabilisieren, daß man ein Gemisch zusetzt, das ein Nitroxyl-Radikal und wenigstens einen Polymerisationsinhibitor aus der Gruppe umfassend Phenothiazin, p-Phenylendiamin, Diphenylamin, Hydrochinon und Hydrochinonmonomethylether, enthält. Ein solches Inhibitorgemisch eigne sich auch zur Minderung der Polymerisationsneigung von (Meth) acrylsäure während mittels stärker Säuren katalysierter Veresterungen der (Meth)acrylsäure. Weiterhin lehrt die JP-A 5/320217, daß zur Minderung der Polymerisationsneigung von (Meth)acrylsäure geeignete Polymerisationsinhibitoren nicht notwendigerweise auch zur Polymerisationsinhibierung deren Ester geeignet sind, was vermutlich darauf zurückzuführen ist, daß der Ester nicht mehr über die Carboxylgruppe verfügt. Dem entsprechend, enthält die JP-A 5/320217 keinerlei Hinweis, daß sich die in der JP-A 5/320217 für (Methlacrylsäure empfohlenen Inhibitorsysteme auch zur Inhibierung der Polymerisationsneigung von (Meth) acrylsäureestern eignen könnten.

Die Aufgabe der vorliegenden Erfindung bestand daher darin, ein Verfahren zur Stabilisierung von als Reinsubstanz oder als Bestandteil von Gemischen, die keine Brönsted-Säure enthalten, deren Brönsted-Säurestärke größer als die Brönsted-Säurestärke von Acrylsäure ist, vorliegenden Estern der (Meth)acrylsäure gegen unerwünschte radikalische Polymerisation durch Zusatz eines ein p-Phenylendiamin enthaltenden Polymerisationsinhibitors zur Verfügung zu stellen, das besser polymeristionsinhibierend wirkt, als die im vorstehend gewürdigten Stand der Technik aufgeführten Verfahren.

Demgemäß wurde ein Verfahren zur Stabilisierung von als Reinsubstanz oder als Bestandteil von Gemischen, die keine Brönsted-Säure enthalten deren Brönsted-Säurestärke größer als die Brönsted-Säurestärke von Acrylsäure ist, vorliegenden Estern der (Meth) acrylsäure gegen unerwünschte radikalische Polymerisation durch Zusatz eines Polymerisationsinhibitors gefunden, das dadurch gekennzeichnet ist, daß man der Reinsubstanz oder dem Gemisch ein Polymerisationsinhibitorgemisch zusetzt, das enthält:

a) wenigstens ein Nitroxyl-Radikal (Inhibitoren a) und
b) wenigstens ein p-Phenylendiamin der allgemeinen Formel (X)

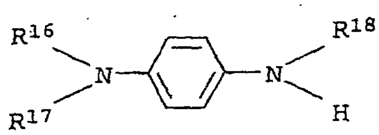

(X)

mit

$R^{16}$, $R^{17}$, $R^{18}$= unabhängig voneinander Alkyl, Aryl, Alkaryl oder Aralkyl mit bis zu 20 C-Atomen,

und ein Rest $R^{16}$, $R^{17}$ oder $R^{18}$ kann auch Wasserstoff sein (Inhibitoren b).

Ein Maß für Brönsted-Säurestärke ist die Gleichgewichtskonstante (Säurekonstante) der Reaktion HA + $H_2O$ $\leftrightarrows$ $A^{\ominus}$+ $H_3O^{\oplus}$ bei einer Temperatur von 25°C und einem Druck von 1 atm. HA Symbolisiert dabei die Brönsted-Säure und $A^{\ominus}$ die zugehörige konjugierte Base (vgl. Christen, Grundlagen der allgemeinen und anorganischen Chemie, Verlag Sauerländer, Aarau, 1973, S. 353/354).

Als erfindungsgemäß geeignete Nitroxyl-Radikale (auch als N-Oxyl-Radikale bezeichnet) kommen insbesondere diejenigen in Betracht, die sich von einem sekundären Amin ableiten, welches keine Wasserstoffatome an den α-C-Atomen trägt (d.h., die N-Oxyl-Gruppen leiten sich von entsprechenden sekundären Aminogruppen ab). Unter diesen eignen sich vor allem jene N-Oxyl-Radikale, die in der EP-A 135280, der älteren Anmeldung DE-A 19651307, der US-A 5,322,912, der US-A 5,412,047, der US-A 4,581,429, der DE-A 1618141, der CN-A 1052847, der US-A 4,670,131, der US-A 5,322,960, der älteren Anmeldung DE-A 19602539, der EP-A 765856 und der JP-A 5/320217 genannt sind.

Solche geeigneten, sich von einem sekundären Amin ableitenden, stabile N-Oxyl-Radikale sind z.B. jene der allgemeinen Formel I

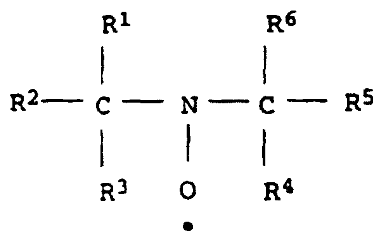

(I),

mit

$R^1$,$R^2$,$R^5$ und $R^6$ = dieselben oder verschiedene gerad- oder verzweigtkettige, gegebenenfalls substituierte Alkylgruppen und

$R^3$ und $R^4$ = dieselben oder verschiedene gerad- oder verzweigtkettige, gegebenenfalls substituierte Alkylgruppen oder

$R^3CNCR^4$ = eine, gegebenenfalls substituierte, zyklische Struktur.

Als erfindungsgemäß geeignete Verbindungen I kommen insbesondere jene in Betracht, die in der EP-A 135 280, der älteren Anmeldung DE-A 19651307, der US-A 5,322,912, der US-A 5,412,047, der US-A 4,581,429, der DE-A 16 18 141, CN-A 1052847, US-A 4,670,131, US-A 5,322,960 sowie der älteren Anmeldung DE-A 19602539 genannt sind.

Beispiele dafür sind jene stabilen N-Oxyl-Radikale der allgemeinen Formel I, bei welchen $R^1$, $R^2$, $R^5$ und $R^6$ für (gleiche oder verschiedene) $C_1$- bis $C_4$-Alkylgruppen wie Methyl-, Ethyl-, n-Propyl-iso-Propyl-, n-Butyl-, iso-Butyl- oder tert.-Butyl-, lineares oder verzweigtes Pentyl-, Phenyl- oder substituierte Gruppen hiervon und $R^3$ und $R^4$ für (gleiche oder verschiedene) $C_1$- bis $C_4$-Alkylgruppen wie Methyl-, Ethyl-, n-Propyl-, iso-Propyl-, n-Butyl-, iso-Butyl- oder tert.-Butyl-, lineares oder verzweigtes Pentyl-, substituierte Gruppen hiervon oder gemeinsam mit CNC die die zyklische Struktur

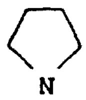 oder 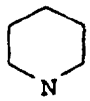 oder 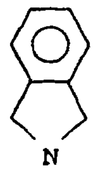 oder 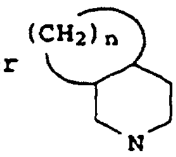 oder 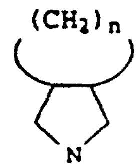

mit n gleich einer ganzen Zahl von 1 bis 10 (häufig 1 bis 6), einschließlich substituierter derartiger zyklischer Strukturen, stehen. Als beispielhafte Vertreter seien 2,2,6,6-Tetramethyl-1-oxyl-piperidin, 2,2,5,5-Tetramethyl-1-oxyl-pyrrolidin und 4-Oxo-2,2,6,6-tetramethyl-1-oxyl-piperidin genannt.

Die N-Oxyl-Radikale I lassen sich aus den entsprechenden sekundären Aminen durch Oxidation, z.B. mit Wasserstoffperoxid, herstellen. In der Regel sind sie als Reinsubstanz darstellbar.

Zu den erfindungsgemäß geeigneten N-Oxyl-Radikalen I zählen insbesondere piperidin- oder pyrrolidin-N-Oxyle und Di-N-Oxyle der nachstehenden allgemeinen Formeln II bis IX:

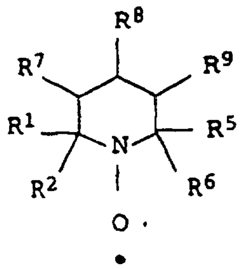
(II),

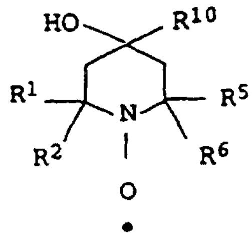
(III),

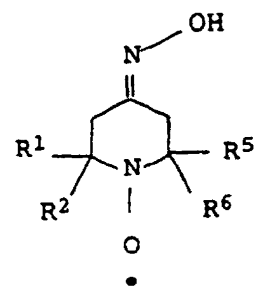
(IV),

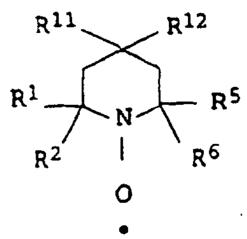
(V),

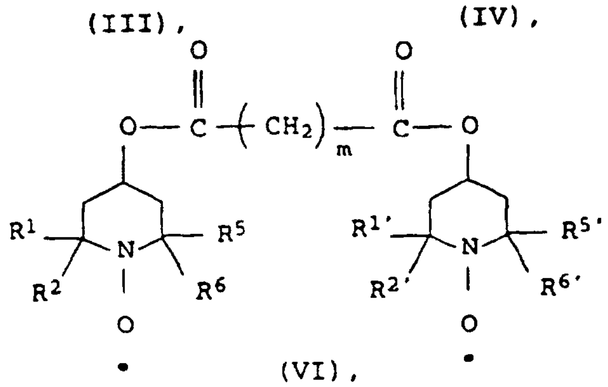
(VI),

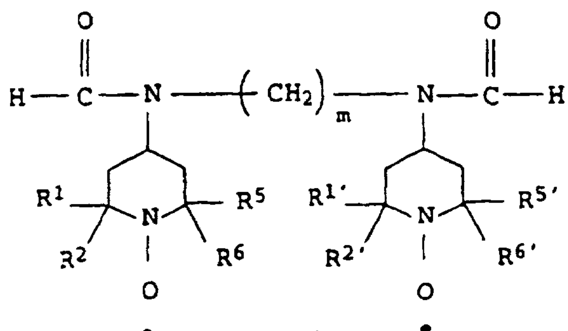
(VII),

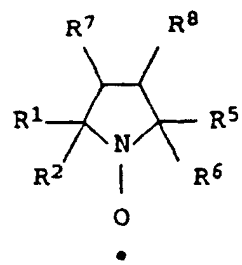
(VIII),

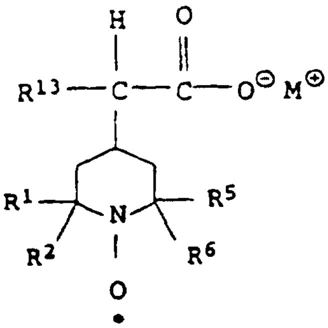
(IX),

mit

m = 2 bis 10,

$R^7,R^8,R^9$ = unabhängig voneinander

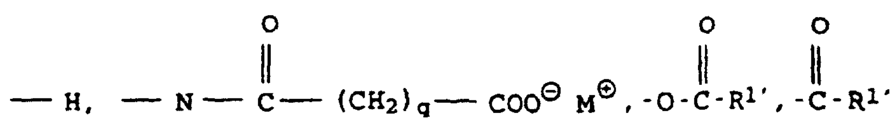

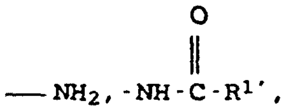, 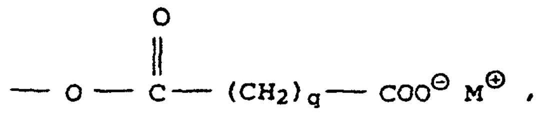,

$—COO^{\ominus} M^{\oplus}$, $—SO_3^{\ominus} M^{\oplus}$, $—PO_3^{\ominus}M^{\oplus}$,
$—O—PO_3^{2\ominus} M_2^{\oplus}$, $—O—SO_3^{\ominus}M^{\oplus}$, —OH,

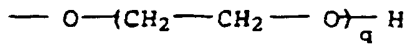

oder

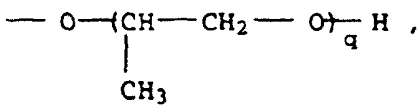

$M^{\oplus}$= ein Wasserstoff- oder ein Alkalimetallion,

q = eine ganze Zahl von 1 bis 10,

$R^{1'}$,$R^{2'}$,$R^{5'}$,$R^{6'}$ = unabhängig voneinander und unabhängig von $R^1$, $R^2$, $R^5$, $R^6$ dieselben Gruppen wie $R^1$,

$R^{10}$ = $C_1$- bis $C_4$-Alkyl, $-CH=CH_2$, $-C\equiv CH$, -CN,

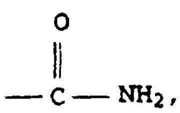

$-COO^{\ominus}M^{\oplus}$, $-COOCH_3$ oder $-COOC_2H_5$,

$R^{11}$ = ein organischer Rest, der wenigstens eine primäre, sekundäre (z.B. $-NHR^1$) oder tertiäre Aminogruppe (z.B. $-NR^1R^2$) oder wenigstens eine Ammoniumgruppe $-N^{\oplus}R^{14}R^{15}R^{16}X^{\ominus}$ aufweist, mit $X^{\ominus}$ = $P^{\ominus}$, $Cl^{\ominus}$, $Br^{\ominus}$, $HSO_4^{\ominus}$, $SO_4^{2\ominus}$, $H_2PO_4^{\ominus}$, $HPO_4^{2\ominus}$ oder $PO_4^{3\ominus}$ und $R^{14}$, $R^{15}$, $R^{16}$ voneinander unabhängige organische Reste (z.B. unabhängig voneinander und unabhängig von $R^1$ dieselben Gruppen wie $R^1$),

$R^{12}$ = unabhängig von $R^{11}$ dieselben Gruppen wie $R^{11}$ oder -H, -OH, $C_1$- bis $C_4$-Alkyl, $-COO^{\ominus}M^{\oplus}$, $-C\equiv CH$,

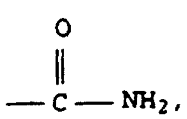, 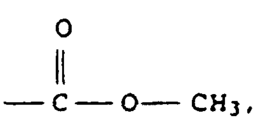, 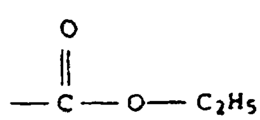

oder hydroxysubstituiertes $C_1$- bis $C_4$-Alkyl (z.B hydroxyethyl oder hydroxypropyl) oder

$R^{11}$, $R^{12}$ = gemeinsam den Sauerstoff einer Carbonylgruppe und

$R^{13}$ = —H, $—CH_3$ oder

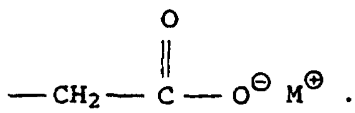

Vorzugsweise ist $R^1 = R^2 = R^5 = R^6 = R^{1'} = R^{2'} = R^{5'} = R^{6'} = -CH_3$.

Als beispielhafte Vertreter erfindungsgemäß geeigneter N-Oxyl-Radikale seien
4-Hydroxy-2,2,6,6-tetramethyl-l-oxyl-piperidin,
4-Hydroxy-2,6-diphenyl-2,6-dimethyl-1-oxyl-piperidin,
4-Carboxy-2,2,6,6-tetramethyl-1-oxyl-piperidin,
4-Carboxy-2,6-diphenyl-2,6-dimethyl-1-oxyl-piperidin,
3-Carboxy-2,2,5,5-tetramethyl-1-oxyl-pyrrolidin,
3-Carboxy-2,5-diphenyl-2,5-dimethyl-1-oxyl-pyrrolidin,
4-Acetyl-2,2,6,6-tetramethyl-1-oxyl-piperidin, N,N'-Bis (1-Oxyl-2,2,6,6-tetramethylpiperidin-4-yl)-N,N'-bis formyl-1,6-diaminohexan und Bis-(1-oxyl-2,2,6,6-tetramethylpiperidin-4-yl)adipat genannt.

Die Herstellung von 3-Carboxy-2,2,5,5-tetramethyl-1-oxyl-pyrrolidin findet sich z.B. in Romanelli, M.; Ottaviani, M.F.; Martini, G.; Kevan, L., JPCH J. Phys. Chem., EN, 93, 1, 1989, S. 317 - 322.

Die Verbindungen (VI) und (VII) können gemäß US-A 4665185 (z.B. Bsp. 7) sowie DE-A 19510184 erhalten werden.

Weitere geeignete beispielhafte Vertreter sind:

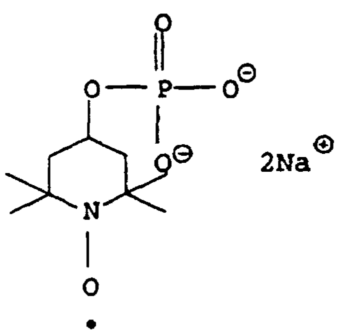

Sunamoto, Junzo; Akiyoshi, Kuzunari, Kihara, Tetsuji; Endo, Masayuki, BCS JA 8, Bull. Chem. Soc. Jpn., EN, 65, 4, 1992, S. 1041 - 1046;

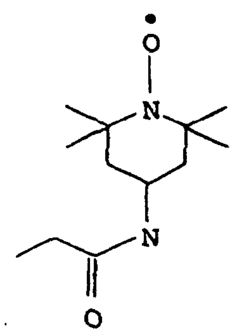

Beilstein Registry Number 6926369 ($C_{11}H_{22}N_3O_2$);

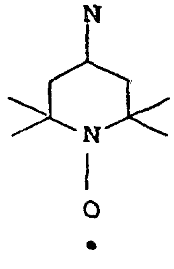

Beilstein Registry Number 6498805 (4-Amino-2,2,6,6-tetramethyl-1-oxyl-piperidin);

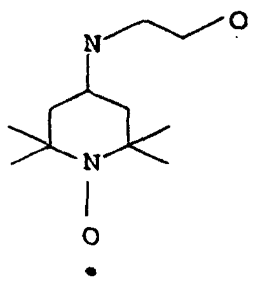

Beilstein Registry Number 6800244 ($C_{11}H_{23}N_2O_2$);

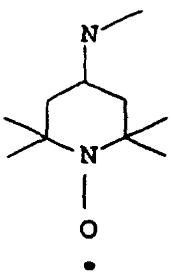

Beilstein Registry Number 5730772 (N-Methyl-4-amino-2,2,6,6-tetra-methyl-1-oxyl-piperidin;

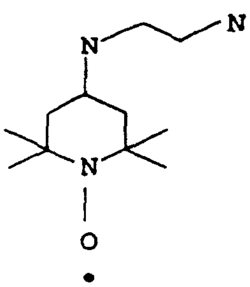

Beilstein Registry Number 5507538 (2,2,6,6-Tetramethyl-4-(2-amino-ethylamino)-1-oxyl-piperidin);

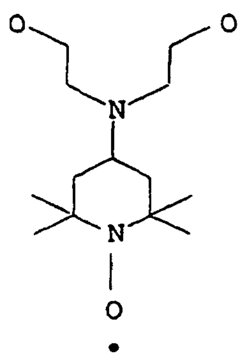

Beilstein Registry Number 4417950 (4<Bis(2-hydroxyethyl)>-amino-2,2,6,6-tetramethyl-1-oxyl-piperi-din);

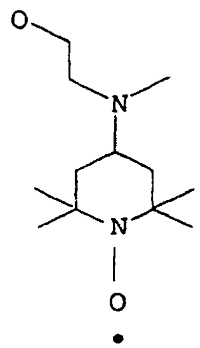

Beilstein Registry Number 4396625 ($C_{12}H_{25}N_2O_2$);

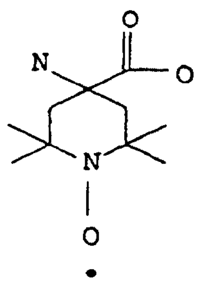

Beilstein Registry Number 4139900 (4-Amino-2,2,6,6-tetramethyl-4-carboxy-1-oxyl-piperidin);

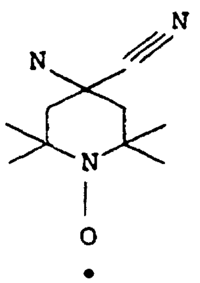

Beilstein Registry Number 4137088 (4-Amino-4-cyano-2,2,6,6-tetramethyl-1-oxyl-piperidin);

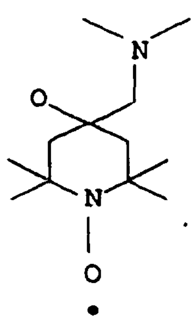

Beilstein Registry Number 3942714 ($C_{12}H_{25}N_2O_2$);

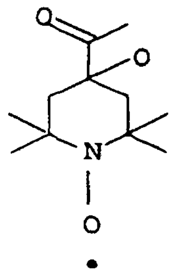

Beilstein Registry Number 1468515 (2,2,6,6-Tetramethyl-4-hydroxy-4-acetyl-1-oxyl-piperidin);

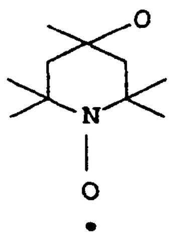

Beilstein Registry Number 1423410 (2,2,4,6,6-Pentamethyl-4-hydroxy-1-oxyl-piperidin);

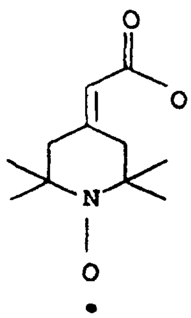

Beilstein Registry Number 6205316 (4-Carboxymethylen-2,2,6,6-tetramethyl-1-oxyl-piperidin);

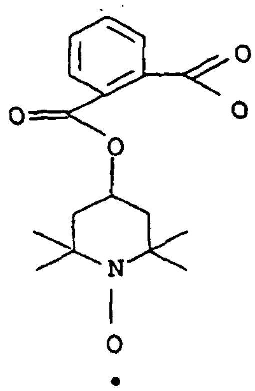

Beilstein Registry Number 1395538 (4-<2-Carboxy-benzoyloxy>-2,2,6,6-tetramethyl-1-oxyl-piperidin);

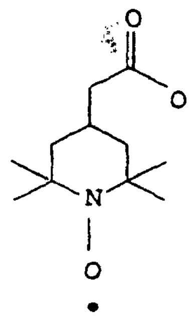

Beilstein Registry Number 3546230 (4-Carboxymethyl-2,2,6,6-tetra-methyl-1-oxyl-piperidin);

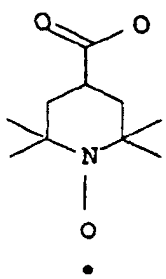

Beilstein Registry Number 3949026 (4-Carboxyl-2,2,6,6-tetra-methyl-1-oxyl-piperidin);

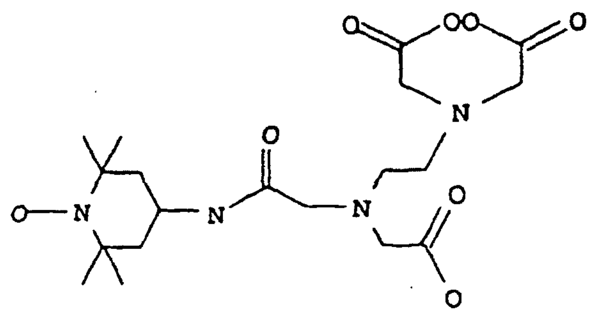

Beilstein Registry Number 4611003 (Ethylendiamintetraessig-säuremono(1-oxy-2,2,6,6-tetramethylpiperidinyl-4-amid);

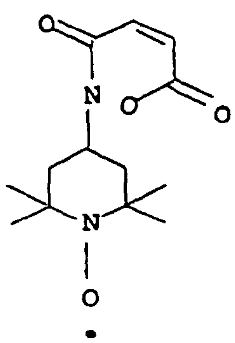

Beilstein Registry Number 5961636 ($C_{13}H_{21}N_2O_4$)

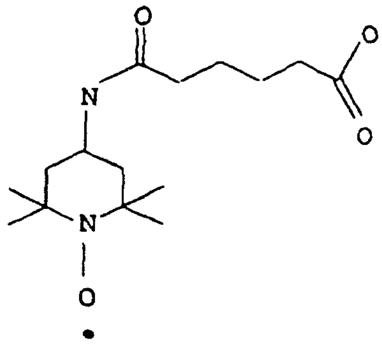

Beilstein Registry Number 5592232 ($C_{15}H_{27}N_2O_4$);

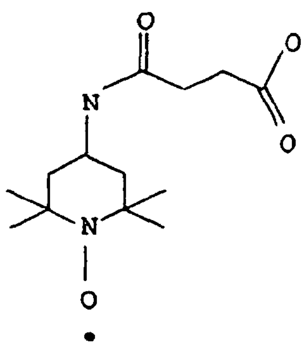

Beilstein Registry Number 5080576 (Bernsteinsäure-N-(2,2,6,6-tetramethyl-1-oxyl-4-piperidinyl)-monoamid);

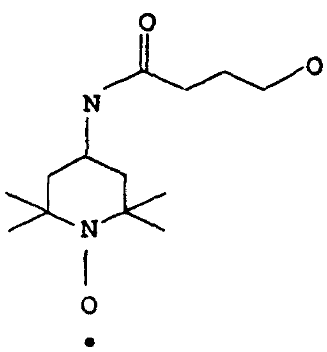

Beilstein Registry Number 5051814 (4-(4-Hydroxybutanoylamino)-2,2,6,6-tetramethyl-1-oxyl-piperidin);

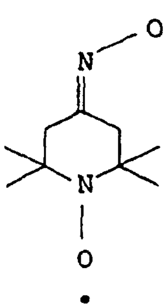

Beilstein Registry Number 4677496 (2,2,6,6-Tetramethyl-4-oximino-1-oxyl-piperidin);

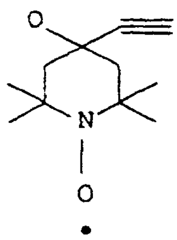

Beilstein Registry Number 1451068 ($C_{11}H_{18}NO_2$);

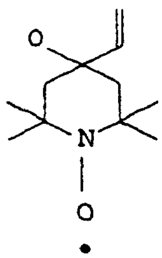

Beilstein Registry Number 1451075 ($C_{11}H_{20}NO_2$);

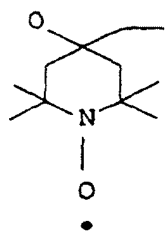

Beilstein Registry Number 1423698 (4-Ethyl-4-hydroxy-2,2,6,6-tetramethyl-1-oxyl-piperidin);

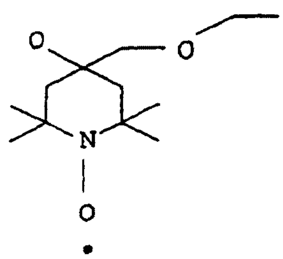

Beilstein Registry Number 5509793 (4-Ethoxymethyl-4-hydroxy-2,2,6,6-tetramethyl-1-oxyl-piperidin);

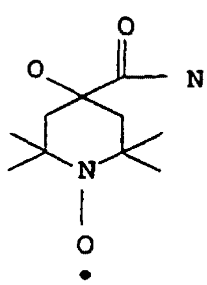

Beilstein Registry Number 3960373 ($C_{10}H_{19}N_2O_3$);

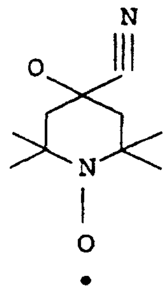

Beilstein Registry Number ($C_{10}H_{17}N_2O_2$);

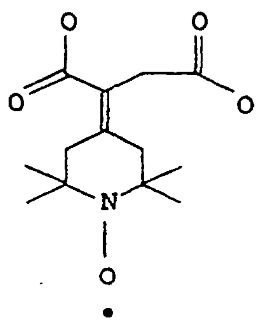

Beilstein Registry Number 3985130 (2,2,6,6-Tetramethyl-1-oxyl-4-piperidyliden)-bernsteinsäure);

Selbstverständlich können erfindungsgemäß auch Gemische von N-Oxyl-Radikalen angewendet werden.

Erfindungsgemäß geeignete p-Phenylendiamine sind solche der allgemeine Formel X

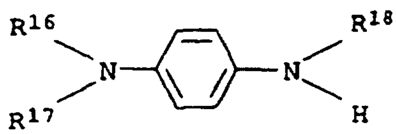

(X)

mit

$R^{16}$, $R^{17}$, $R^{18}$= unabhängig voneinander Alkyl, Aryl, Alkaryl oder Aralkyl mit bis zu 20 C-Atomen,

und ein Rest $R^{16}$, $R^{17}$ oder $R^{18}$ kann auch Wasserstoff sein.

Insbesondere eignen sich Verbindungen X mit $R^{16}$, $R^{17}$, $R^{18}$= unabhängig voneinander Methyl, Ethyl, Propyl, iso-Propyl, iso-Butyl, sek.-Butyl, n-Butyl, Pentyl, Phenyl oder Naphthyl. Als Beispiele für geeignete Verbindungen X seien genannt: N.N'-Bis-sek.butylp-phenylendiamin, N-Phenyl-N' -isopropyl-phenylendiamin, N-Naphthyl-N'-sek-butyl-p-phenylendiamin, N,N,N'-Trimethylp-phenylendiamin, N,N,N' -Triethyl-p-phenylendiamin, N,N-Dimethyl-p-phenylendiamin, N,N-Diethyl-p-phenylendiamin, N-Phenyl-N',N'-dimethyl-p-phenylendiamin, N-Phenyl-N',N' -diethylp-phenylendiamin, N-Phenyl-N',N'-dipropyl-p-phenylendiamin, N-Phenyl-N',N'-di-n-butyl-p-phenylendiamin, N-Phenyl-N',N'-di-sek.butyl-p-phenylendiamin, N-Phenyl-N'-methyl-N'-ethyl-p-phenylendiamin, N-Phenyl-N'-methyl-N'-propyl-p-phenylendiamin, N-Phenyl-N'-methyl-p-phenylendiamin, N-Phenyl-N' -ethyl-p-phenylendiamin, N-Phenyl-N'-propyl-p-phenylendiamin, N-Phenyl-N'-isopropylp-phenylendiamin, N-Phenyl-N'-butyl-p-phenylendiamin, N-Phenyl-N'-isobutyl-p-phenylendiamin, N-Phenyl-N'-sek-butyl-p-phenylendiamin, N-Phenyl-N'-tert.-butyl-p-phenylendiamin, N-Phenyl-N'n-pentyl-p-phenylendiamin, N-Phenyl-N' -n-hexyl-p-phenylendiamin, N-Phenyl-N'-(1-methylhexyl)-p-phenylendiamin, N-Phenyl-N'-(1,3-dimethylbutyl)-p-phenylendiamin, N-Phenyl-N'-(1,4-dimethylpentyl) -p-phenylendiamin. Selbstverständlich können erfindungsgemäß auch Gemische von p-Phenylendiaminen eingesetzt werden. Als solche Gemische kommen insbesondere die in der WO 92/01665 empfohlenen p-Phenylendiamingemische in Betracht.

In zweckmäßiger Weise enthält das erfindungsgemäß zuzusetzende Inhibitorgemisch, bezogen auf die darin enthaltene Gesamtmenge an Inhibitoren a) und Inhibitoren b), 1 bis 75 Gew.-% an Inhibitoren a) und 25 bis 99 Gew.-% an Inhibitoren b).

D.h., erfindungsgemä3 zuzusetzende Inhibitorgemische sind auch solche, die, bezogen auf die darin enthaltene Gesamtmenge an Inhibitoren a) und Inhibitoren b), 5 bis 50 Gew.-% (oder 5 bis 25 Gew.-%) an Inhibitoren a) und 50 bis 95 Gew.-% (oder 75 bis 95 Gew.-%) an Inhibitoren b) enthalten.

Bezogen auf das die zu stabilisierenden Ester enthaltende Gemisch gewährleistet in der Regel ein Zusatz an Inhibitoren a) und Inhibitoren b) von insgesamt 10 bis 5000 Gew. ppm, häufig 10 bis 1000 Gew. ppm, befriedigende Stabilität. Letzteres gilt auch für bei destillativer Behandlung von (Meth)acrylsäureestern üblichen Temperaturen von 50 bis 200°C, häufig 60°C bis 160°C.

Als zu stabilisierende (Meth)acrylsäureester kommen insbesondere Ester aus (Meth)acrylsäure und ein- oder mehrwertigen Alkanolen in Betracht. Dies gilt insbesondere dann, wenn die ein oder mehrwertigen Alkanole 1 bis 20 C-Atome, oder 1 bis 12 C-Atome oder 1 bis 8 C-Atome aufweisen. Beispielhafte Vertreter solcher Ester sind z.B. Methylacrylat, Ethylacrylat, n-Butylacrylat, iso-Butylacrylat, tert.-Butylacrylat, 2-Ethylhexylacrylat, Methylmethacrylat, Ethylmethacrylat, n-Butylmethacrylat und tert.-Butylmethacrylat.

Das erfindungsgemäße Verfahren eignet sich vor allem zur Stabilisierung von (Meth)acrylsäureestern (insbesondere der vorgenannten beispielhaften Vertreter) bei deren destillativer (o. rektifikativer) Abtrennung aus Produktgemischen, wie sie bei der säurekatalysierten Veresterung von (Meth)acrylsäure mit Alkoholen, insbesondere Alkanolen (insbesondere $C_1$- bis $C_{12}$- bzw. $C_1$- bis $C_8$-Alkanolen) nach Abtrennung des Säurekatalysators vorliegen.

Es eignet sich aber auch zur Stabilisierung von vorgenannte (Meth)acrylsäureester enthaltenden Gemischen, die weder eine Brönsted-Säure deren Brönsted-Säurestärke größer als die Brönsted-Säurestärke von Acrylsäure ist, noch Acryl- oder Methacrylsäure selbst enthalten. Solche (Meth)acrylsäureester enthaltenden Gemische bilden beispielsweise vorgenannte Veresterungsproduktgemische nach Abtrennung des Säurekatalysators sowie nach Abtrennung der überschüssigen (Meth)acrylsäure (in der Regel beträgt der (Meth)acrylsäuregehalt vorgenannter Gemische nach Abtrennung der überschüssigen (Meth)acrylsäure ≤ 1 Gew.-%, häufig ≤ 100 Gew.ppm und vielfach ≤ 50 Gew. ppm bezogen auf das Gemisch; vielfach gelingt die (Meth)acrylsäureabtrennung quantitativ).

Solche an Säure freie Veresterungsproduktgemische enthalten in der Regel 80 bis 95 Gew.-% (Meth)acrylsäureester, 1 bis 10 Gew.-% zur Veresterung eingesetzter Alkohol, gegebenenfalls ein zur Veresterung eingesetztes organisches Lösungsmittel und geringe Mengen tiefer und höher als der (Meth)acrylsäureester siedender Nebenprodukte (z.B. Wasser, Ether oder Michael-Addukte). Typische Wassergehalte liegen bei 0,5 bis 3 Gew.-%, bezogen auf das Veresterungsproduktgemisch.

Die erfindungsgemäß zuzusetzenden Inhibitorgemische weisen in der Regel auch eine ausreichende Wasserlöslichkeit auf, so daß sie auch in solchen (Meth) acrylsäureester enthaltenden Gemischen anwendbar sind, die neben einer organischen zusätzlich eine wäßrige Phase aufweisen.

Es überrascht, daß Gemische aus Inhibitoren a) und Inhibitoren b) eine ausgeprägte synergistische Wirksamkeit im Hinblick auf die Stabilisierung von (Meth)acrylsäureestern aufweisen.

Die Stabilisierung eines einer Destillation (Rektifikation) unterworfenen (Meth)acrylsäureester enthaltenden Gemisches kann in einfacher Weise so erfolgen, daß man die erfindungsgemäß zuzusetzenden Inhibitoren dem Gemisch bereits vor der Destillation (Rektifikation) zugibt. Die Zugabe kann aber auch in den Zulauf zur Destillations (Rektifikations)kolonne erfolgen. In beiden vorgenannten Fällen muß zusätzlich zur Stabilisierung der Kolonne Inhibitorzugabe auf den Kopf der Kolonne erfolgen. Selbstverständlich kann auch die Gesamtstabilisierung ausschließlich über eine Inhibitorzugabe auf den Kolonnenkopf vorgenommen werden. Selbstredend können beim erfindungsgemäßen Verfahren die Inhibitoren a) und Inhibitoren b) zeitlich nacheinander, simultan oder auch bereits vorgemischt zugesetzt werden. Das vorgenannte gilt auch für die anderen Inhibitoren, falls das Inhibitorgemisch solche umfaßt. Selbstverständlich kann das erfindungsgemäß zuzusetzende Inhibitorgemisch neben Polymerisationsinhibitoren a) und b) noch andere Polymerisationsinhibitoren wie Phenothiazin oder phenolische Verbindungen wie Hydrochinon oder dessen Methylether enthalten. Mit Vorteil wird es jedoch keine solchen zusätzlichen Polymerisationsinhibitoren enthalten. Günstig ist, daß das erfindungsgemäß zu verwendende Inhibitorgemisch seine Wirksamkeit auch im Beisein von molekularem Sauerstoff entfaltet, weshalb die die (Meth)acrylsäureester enthaltenden Vorrichtungen, wie z.B. Destillations(Rektifikations)kolonnen, bei Anwendung des erfindungsgemäßen Verfahrens häufig von molekularem Sauerstoff oder Luft durchströmt werden.

Die Wirksamkeit von Inhibitorgemischen wird in der Regel so bestimmt, daß man dem zu stabilisierenden Gemisch bei Raumtemperatur (25°C) das Inhibitorgemisch zusetzt. Anschließend wird auf eine erhöhte Temperatur (in typischer Weise im Bereich von 50 bis 150°C liegend) erwärmt und dann die Zeitdauer bis zum Beginn der Polymerisation (onset time) ermittelt. Da die Polymerisation exotherm verläuft, macht sich ihr Einsetzen durch einen Temperatursprung bemerkbar (vgl. Bockstahler u.a., Ind. & Eng. Chem., 50(10), 1581).

## Beispiele und Vergleichsbeispiele

### Beispiel 1

50 ml eines Gemisches aus 90,9 Gew.-% n-Butylacrylat, 4 Gew.-% n-Butanol, 0,8 Gew.-% Di-n-butylether,

0,5 Gew.-% n-Butylacetat, 0,9 Gew.-% Wasser und 2,9 Gew.-% Butoxypropionsäurebutylester wurden in einem 100 ml Rundkolben, der mit einem Rückflußkühler und einem mit einem Schreiber verbundenen Thermoelement ausgerüstet war, mit X Gew.ppm von verschiedenen Polymerisationsinhibitoren versetzt.

Dann wurde das Gemisch mittel eines thermostatisierten Ölbads auf 80°C erhitzt. Tabelle 1 weist die für die verschiedenen Inhibitorsysteme ermittelten onset-Zeiten aus.

Tabelle 1

| Inhibitor | Inhibitormenge (Gew.ppm) | onset-time (h) |
|---|---|---|
| - | 0 | 4 |
| Phenothiazin | 10 | 200 |
| Hydrochinon | 10 | 175 |
| N,N'-Bis-sek.butyl-p-phenylendiamin (KBPD) | 10 | 370 |
| p-Nitrosophenol | 10 | 135 |
| 2,2,6,6-Tetramethyl-1-oxyl-piperidin (TEMPO) | 10 | 760 |
| 4-Acetoxy-2,2,6,6-tetramethyl-1-oxyl-piperidin (4-Acetoxy-TEMPO) | 10 | 690 |
| KBPD<br>TEMPO | 5<br>5 | 1130 |
| KBPD<br>P-Nitrosophenol | 5<br>5 | 850 |
| Phenothiazin<br>TEMPO | 5<br>5 | 480 |

Beispiel 2 (nicht erfindungsgemäß)

Ein auf dem Weg der direkten sauren Veresterung von Acrylsäure mit n-Butanol gewonnenes n-Butylacrylat-Rohestergemisch hatte nach Abtrennung des Säurekatalysators und der überschüssigen Acrylsäure folgende Zusammensetzung:

| | |
|---|---|
| n-Butylacrylat | 88 Gew.-% |
| n-Butanol | 4,7 Gew.-% |
| Di-n-butylether | 0,7 Gew.-% |
| n-Butylacetat | 0,5 Gew.-% |
| Butoxypropionsäurebutylester | 2,9 Gew.-% |
| Wasser | 1,1 Gew.-% |

Aus diesem Gemisch wurden in einer ersten Rektifikation die leichter als n-Butylacrylat siedenden Komponenten und aus dem dabei anfallenden Sumpf in einer zweiten Rektifikationskolonne n-Butylacrylat von den schwerer als n-Butylacrylat siedenden Komponenten abgetrennt.

Der Zulauf (30 $m^3$/h) des Rohestergemisches zur ersten Rektifikationskolonne (60 dual-flow Böden, natürlicher Umlaufverdampfer) erfolgte auf den 49ten Boden. Die Sumpftemperatur betrug 110°C (Sumpfdruck=300 mbar), die Kopftemperatur betrug 86°C (Kopfdruck: 150 mbar) und der Rücklauf betrug 20 $m^3$/h. Die über Kopf ausgeschleuste Destillatmenge betrug 5,6 $m^3$/h. Die Stabilisierung erfolgte durch Zugabe von 100 Gew.ppm KBPD und 20 Gew.ppm p-Nitrosophenol in den Rücklauf (Gew.ppm auf die Rücklaufmenge bezogen). Aus dem Sumpf der ersten Rektifikationskolonne, der die Schwersieder und n-Butylacrylat enthielt, wurde in einer zweiten Rektifikationskolonne (30 dual-flow Böden, natürlicher Umlaufverdampfer) n-Butylacrylat mit einer Reinheit von 99,8 Gew.-% über den Kopf der Kolonne abgetrennt (21,2 $m^3$/h). In der zweiten Rektifikationskolonne betrug die Sumpftemperatur 103°C (Sumpfdruck: 150 mbar), die Kopf temperatur 80°C (Kopfdruck: 100 mbar) und das Rücklaufvolumenverhältnis 1:4. Der Zulauf der Sumpfflüssigkeit aus der ersten Rektifikationskolonne in die zweite Rektifikationskolonne erfolgte auf den zehnten Boden. Zur Stabilisierung der zweiten Kolonne wurden selbiger über den Rückfluß 200 Gew.ppm Monomethylether des Hydrochinon (Gew.ppm auf die Rücklaufmenge bezogen) zugefügt.

Auch nach 25 Tagen Laufzeit der Rektifikationseinheit bestand noch keine Notwendigkeit, selbige abzuschalten.

Vergleichsbeispiel 1

Es wurde wie in Beispiel 2 verfahren, die Stabilisierung der ersten Rektifikationskolonne erfolgte jedoch ausschließlich mittels 150 Gew.ppm KBPD (Gew.ppm auf den Rücklauf bezogen). Infolge von Verstopfung der Verdampferrohre und Belagbildung auf den Kolonnenböden (jeweils der ersten Rektifikationskolonne) mußte die Rektifikationseinheit nach 5 Tagen abgestellt werden.

Beispiel 3

Es wurde wie in Beispiel 2 verfahren, anstelle von 20 Gew.ppm p-Nitrosophenol wurden jedoch 10 Gew.ppm 4-Hydroxy-2,2,6,6-tetramethyl-1-oxyl-piperidin verwendet. Auch nach 25 Tagen Laufzeit der Rektifikationseinheit bestand noch keine Notwendigkeit, selbige abzuschalten.

Beispiel 4

Es wurde wie in Beispiel 2 verfahren, anstelle von 20 Gew.ppm p-Nitrosophenol wurden jedoch 20 Gew.ppm N,N'-bis-(1-Oxyl-2,2, 6,6-tetramethylpiperidin-4-yl) -N,N'-bis-formyl-1,6-diaminohexan eingesetzt. Auch nach 25 Tagen Laufzeit der Rektifikationseinheit bestand noch keine Notwendigkeit, selbige abzuschalten.

Vergleichsbeispiel 2

Es wurde wie in Beispiel 2 verfahren, die Stabilisierung der ersten Rektifikationskolonne erfolgte jedoch mit 150 Gew.ppm Hydrochinonmonomethylether und 10 Gew.ppm 4-Hydroxy-2,2,6,6-tetramethyl-1-oxyl-piperidin. Nach 6 Tagen mußte die Rektifikation wegen Belagbildung auf den Kolonnenböden und in den Verdampferrohren abgestellt werden.

## Patentansprüche

1. Verfahren zur Stabilisierung von als Reinsubstanz oder als Bestandteil von Gemischen, die keine Brönsted-Säure enthalten deren Brönsted-Säurestärke größer als die Brönsted-Säurestärke von Acrylsäure ist, vorliegenden Estern der (Meth)acrylsäure gegen unerwünschte radikalische Polymerisation durch Zusatz eines Polymerisationsinhibitors, das **dadurch gekennzeichnet ist, daß** man der Reinsubstanz oder dem Gemisch Polymerisationsinhibitoren zusetzt, die enthalten:

   a) wenigstens ein Nitroxyl-Radikal (Inhibitoren a)
   und

   b) wenigstens ein p-Phenylendiamin der allgemeinen Formel (X)

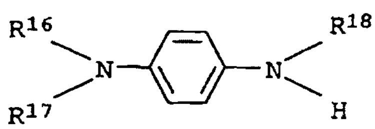

(X)

   mit

   $R^{16}$, $R^{17}$, $R^{18}$= unabhängig voneinander Alkyl, Aryl, Alkaryl oder Aralkyl mit bis zu 20 C-Atomen,

   und ein Rest $R^{16}$, $R^{17}$ oder $R^{18}$ kann auch Wasserstoff sein (Inhibitoren b).

2. Verfahren nach Anspruch 1, **dadurch gekennzeichnet, daß** das p-Phenylendiamin ausgewählt ist aus der Gruppe N,N'-Bis-sek.butyl-p-phenylendiamin, N-Phenyl-N'-isopropyl-phenylendiamin, N-Naphthyl-N'-sek-butyl-p-phenylendiamin, N,N,N'-Trimethylp-phenylendiamin, N,N,N' -Triethyl-p-phenylendiamin, N,N-Dimethyl-p-phenylendiamin, N,N-Diethyl-p-phenylendiamin, N-Phenyl-N',N'-dimethyl-p-phenylendiamin, N-Phenyl-N',N'-diethylp-phenylendiamin, N-Phenyl-N',N'-dipropyl-p-phenylendiamin, N-Phenyl-N',N'-di-n-butyl-p-phenylendiamin, N-Phenyl-N', N'di-sek.-butyl-p-phenylendiamin, N-Phenyl-N'-methyl-N'-ethylp-phenylendiamin, N-Phenyl-N'-methyl-N'-propyl-p-phenylendiamin, N-Phenyl-N'-methyl-p-phenylendiamin, N-Phenyl-N'ethyl-p-phenylendiamin, N-Phenyl-N'-propyl-p-phenylendiamin, N-Phenyl-N'-isopropyl-p-phenylendiamin, N-Phenyl-N'-butylp-phenylendiamin, N-Phenyl-N'-isobutyl-p-phenylendiamin, N-Phenyl-N'-sek-butyl-p-phenylendiamin, N-Phenyl-N'-tert.butyl-p-phenylendiamin,N-Phenyl-N'-n-pentyl-p-phenylendiamin,N-Phenyl-N'-n-hexyl-p-phenylendiamin,N-Phenyl-N'-(1-methylhexyl)-p-phenylendiamin, N-Phenyl-N'-(1,3-dimethylbutyl)-p-phenylendiamin, N-Phenyl-N'-(1,4-dimethylpentyl)-p-phenylendiamin.

3. Verfahren nach Anspruch 1 oder 2, **dadurch gekennzeichnet, daß** das den (Meth)acrylsäureester enthaltende Gemisch <1 Gew.-% (Meth)acrylsäure enthält.

4. Verfahren nach einem der Ansprüche 1 bis 3, **dadurch gekennzeichnet, daß** das den (Meth)acrylsäureester enthaltende Gemisch Wasser enthält.

5. Verfahren nach einem der Ansprüche 1 bis 4, **dadurch gekennzeichnet, daß** der (Meth)acrylsäureester ein solcher eines $C_1$bis $C_8$-Alkanols ist.

6. Verfahren nach einem der Ansprüche 1 bis 5, **dadurch gekennzeichnet, daß** als Polymerisationsinhibitoren nur Inhibitoren a) und Inhibitoren b) zugesetzt werden.

## Claims

1. A method of stabilizing esters of (meth)acrylic acid, which are present as a pure substance or as a constituent of mixtures which comprise no Brönsted acid whose Brönsted acid strength is greater than the Brönsted acid strength of acrylic acid, against unwanted free-radical polymerization, by addition of a polymerization inhibitor, which comprises adding to the pure substance or to the mixture polymerization inhibitors which comprise:

   a) at least one nitroxyl radical (inhibitors a)
   and
   b) at least one p-phenylenediamine of the formula (X)

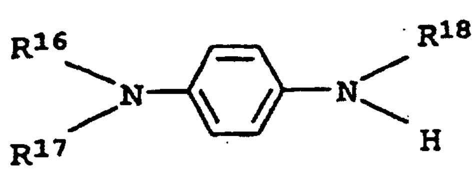

(X)

   where

   $R^{16}$, $R^{17}$, $R^{18}$ = independently of one another alkyl, aryl, alkaryl or aralkyl having up to 20 carbons,

   and one radical $R^{16}$, $R^{17}$ or $R^{18}$ can also be hydrogen (inhibitors b).

2. A method as claimed in claim 1, wherein the p-phenylenediamine is selected from the group consisting of N,N'-bis-sec-butyl-p-phenylenediamine, N-phenyl-N'-isopropylphenylenediamine, N-naphthyl-N'-sec-butyl-p-phenylenediamine, N,N,N'-trimethyl-p-phenylenediamine, N,N,N'-triethyl-p-phenylenediamine, N,N-dimethyl-p-phenylenediamine, N,N-diethyl-p-phenylenediamine, N-phenyl-N',N'-dimethyl-p-phenylenediamine, N-phenyl-N',N'-diethyl-p-phenylenediamine, N-phenyl-N',N'-dipropyl-p-phenylenediamine, N-phenyl-N',N'-di-n-butyl-p-phenylenediamine, N-phenyl-N',N'-di-sec-butyl-p-phenylenediamine, N-phenyl-N'-methyl-N'-ethyl-p-phenylenediamine, N-phenyl-N'-methyl-N'-propyl-p-phenylenediamine, N-phenyl-N'methyl-p-phenylenediamine, N-phenyl-N'-ethyl-p-phenylenediamine, N-phenyl-N'-propyl-p-phenylenediamine, N-phenyl-N'-isopropyl-p-phenylenediamine, N-phenyl-N'-butyl-p-phenylenediamine, N-phenyl-N'-isobutyl-p-phenylenediamine, N-phenyl-N'-sec-butyl-p-phenylenediamine, N-phenyl-N'-tert-butyl-p-phenylenediamine, N-phenyl-N'-n-pentyl-p-phenylenediamine, N-phenyl-N'-n-hexyl-p-phenylenediamine, N-phenyl-N'-(1-methylhexyl)-p-phenylenediamine, N-phenyl-N'-(1,3-dimethylbutyl)-p-phenylenediamine and N-phenyl-N'-(1,4-dimethylpentyl)-p-phenylenediamine.

**3.** A method as claimed in claim 1 or 2, wherein the mixture comprising the (meth)acrylic ester comprises <1% by weight of (meth)acrylic acid.

**4.** A method as claimed in any one of claims 1 to 3, wherein the mixture comprising the (meth)acrylic ester comprises water.

**5.** A method as claimed in any one of claims 1 to 4, wherein the (meth)acrylic ester is that of a $C_1$-$C_8$-alkanol.

**6.** A method as claimed in any one of claims 1 to 5, wherein the polymerization inhibitors added are only inhibitors a) and inhibitors b).

## Revendications

**1.** Procédé de stabilisation, vis-à-vis d'une polymérisation par voie radicalaire indésirable, d'esters d'acide (méth) acrylique se présentant en tant que substances pures ou que constituants de mélanges ne contenant pas d'acides de Brönstedt dont la force d'acidité selon Brönstedt est supérieure à la force d'acidité selon Brönstedt de l'acide acrylique, par addition d'un inhibiteur de polymérisation, **caractérisé en ce que** l'on ajoute à la substance pure ou au mélange des inhibiteurs de polymérisation contenant:

a) au moins un radical nitroxyle (inhibiteurs a)
et
b) au moins une p-phénylènediaminé de la formule générale (X)

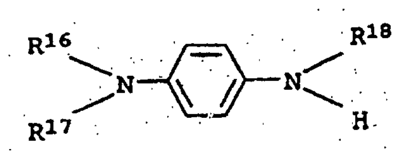

(X)

avec

$R^{16}$, $R^{17}$, $R^{18}$ = indépendamment les uns des autres, des radicaux alkyle, aryle, alkaryle ou aralkyle comportant jusqu'à 20 atomes de C

et un reste $R^{16}$, $R^{17}$ ou $R^{18}$ pouvant également être de l'hydrogène (inhibiteurs b).

**2.** Procédé selon la revendication 1, **caractérisé en ce que** la p-phénylènediamine est choisie dans le groupe formé par la N,N'-bis-sec.-butyl-p-phénylènediamine, la N-phényl-N'-isopropyl-phénylène-diamine, la N-naphthyl-N'-sec.-butyl-p-phénylènediamine, la N,N,N'-triméthyl-p-phénylènediamine, la N,N,N'-triéthyl-p-phénylène-diamine, la N,N-diméthyl-p-phénylènediamine, la N,N-diéthyl-p-phénylènediamine, la N-phényl-N',N'-diméthyl-p-phénylènediamine, la N-phényl-N',N'-diéthyl-p-phénylènediamine, la N-phényl-N',N'-dipropyl-p-phénylène-diamine, la N-phényl-N',N'-di-n-butyl-p-phénylènediamine, la N-phényl-N',N'-di-sec.-butyl-p-phénylènediamine, la N-phényl-N'-méthyl-N'-éthyl-p-phénylène-diamine, la N-phényl-N'-méthyl-N'-propyl-p-phénylène-diamine, la N-phényl-N'-méthyl-p-phénylènediamine,la N-phényl-N'-éthyl-p-phénylènediamine, la N-phényl-N'-propyl-p-phénylènediamine, la N-phényl-N'-isopropyl-p-phénylènediamine, la N-phényl-N'-butyl-p-phénylènediamine, la N-phényl-N'-isobutyl-p-phénylènediamine, la N-phényl-N'-sec.-butyl-p-phénylènediamine, la N-phényl-N'-tert.-butyl-p-phénylènediarnin, la N-phényl-N'-n-pentyl-p-phénylènediamine, la N-phényl-N'-n-hexyl-p-phénylènediamine, la N-phényl-N'-(1-méthylhexyl)-p-phénylènediamine,laN-phényl-N'-(1,3-diméthylbutyl)-p-phénylène-diamine,laN-phényl-N'-(1,4-diméthylpentyl)-p-phénylènediamine.

**3.** Procédé selon la revendication 1 ou 2, **caractérisé en ce que** le mélange contenant l'ester d'acide (méth)acrylique contient moins de 1 % en poids d'acide (méth)acrylique.

**4.** Procédé selon l'une quelconque des revendications 1 à 3, **caractérisé en ce que** le mélange contenant l'ester

d'acide (méth)acrylique contient de l'eau.

**5.** Procédé selon l'une quelconque des revendications 1 à 4, **caractérisé en ce que** l'ester d'acide (méth)acrylique est celui d'un alcanol en $C_1$ à $C_8$.

**6.** Procédé selon l'une quelconque des revendications 1 à 5, **caractérisé en ce que** l'on n'ajoute comme inhibiteurs de polymérisation que des inhibiteurs a) et des inhibiteurs b).